# EUROPEAN PATENT SPECIFICATION

(11) **EP 1 960 416 B1**
(45) Date of publication and mention of the grant of the patent: **31.12.2014**
(21) Application number: 06783444.0
(22) Date of filing: 28.07.2006
(51) Int. Cl.: C07J 9/00

(54) **PURIFICATION PROCESS FOR CHENODEOXYCHOLIC ACID**
VERFAHREN ZUR AUFREINIGUNG VON CHENODESOXYCHOLSÄURE
PROCÉDÉ DE PURIFICATION POUR L'ACIDE CHÉNODÉSOXYCHOLIQUE

(30) Priority: 12.12.2005 KR 20050121605
(43) Date of publication of application: 27.08.2008
(73) Proprietor: DAEWOONG PHARMACEUTICAL CO., LTD., Sungnam-si Kyunggi-do 462-120 (KR); DAEWOONG BIO INC., Hyangnam-eup, Hwaseong-si Gyeonggi-do 445-937 (KR)
(72) Inventor: KIM, Tae Yi, Seoul 135-715 (KR); KIM, Young Soo, Seoul 135-715 (KR); LIM, Young Mook, Seoul 140-831 (KR); KIM, Wol Young, Gyeonggi-do 463-851 (KR); YOON, Yeon Jung, Gyeonggi-do 440-726 (KR); JIN, Yong Suk, Gyeonggi-do 426-855 (KR); LEE, Byung Goo, Gyeonggi-do 440-818 (KR); CHOI, Soo Jin, Seoul 135-715 (KR); LEE, Sung Jae, Seoul 135-715 (KR)
(74) Representative: Wichmann, Hendrik
(86) International application number: PCT/KR2006/002972
(87) International publication number: WO 2007/069814

(56) References cited:
- EP-A- 0 386 538
- WO-A-2007/078039
- CN-A- 1 528 779
- FR-A1- 2 429 224
- JP-A- 03 227 998
- JP-A- 60 181 096
- US-A- 3 919 266
- US-A- 4 186 143
- ZHANG ET AL.: 'Isolation and purification of chenodeoxycholic acid from pig bile' SHENGWU HUAXUE YU SHENGWU WULI JINZHAN no. 4, 1987, pages 68 - 71, XP008125239

## Description

### TECHNICAL FIELD

The present invention relates to a process for purifying chenodeoxycholic acid (3α,7α-dihydroxy-5β-cholic acid). In particular, the present invention relates to a process for purifying chenodeoxycholic acid from low grade chenodeoxycholic acid mixture contained in swine bile solid, with high yield and purity.

### BACKGROUND ART

Chenodeoxycholic acid is generally contained in bile of cow, swine, bear, or poultry such as chicken or goose, as well as in bile of human. Chenodeoxycholic acid is used as starting material for the preparation of ursodeoxycholic acid which is effective to alleviate biliary system diseases, hyperlipidemia, cholelithiasis, and chronic liver diseases, and a typical process for preparing ursodeoxycholic acid known in the art is as follows.

A typical process for preparing chenodeoxycholic acid comprises the steps of: esterifying cholic acid (3α,7α,12α-trihydroxy cholic acid) with methyl; protecting the hydroxyl group of 3α and 7α position by acetylating them with anhydrous acetic acid; oxidizing the hydroxyl group of 12α position to carbonyl group by using chromic acid, and then removing the carbonyl group by Wolff-kichner reduction reaction; hydrolyzing and deprotecting the obtained product to yield chenodeoxycholic acid. The above process requires the reaction to be maintained at a high temperature of more than 200°C, and the supply of raw material may be interrupted by bovine spongiform encephalopathy, etc.

Bile of poultry contains chenodeoxycholic acid, lithocholic acid, and a small amount of cholic acid. Thus, the process for separating chenodeoxycholic acid from poultry is well known in the art, but is not economically reasonable due to the supply decrease of raw material and low yield [see, Windhaus et al, I Physiol. Chem., 140, 177~185 (1924)].

US Patent No. 4,186,143 disclosed a process for purely separating and purifying chenodeoxycholic acid from chenodeoxycholic acid mixture derived from natural swine bile. This process comprises the major steps of: pre-treatment to remove 3α-hydroxy-6-oxo-5β-cholic acid by saponification of bile; esterification of bile acid; acetylation of bile acid ester; removal of intermediate product by using non-polar organic solvent; crystallization of acetylated ester of formula I; deprotection; and production of the compound of formula I by using crystallization in organic solvent. However, this patent does not describe HPLC content for acetylated ester of formula I, and the purity of the final product is very low since the specific rotatory power is [α]_{D}²⁵ +13.8° (c=1, CHCl₃), and the melting point is 119~121°C [STD: [α]_{D}²⁵ +15.2°(c=1, CHCl₃), melting point 127~129°C]. Also, the crystallization for purifying the final product requires a very long time (i.e., 16-48 hours), and the entire process is complex as eight (8) steps. Thus, when purifying the compound of formula I by using the above process, the yield of the final product becomes low, and the reaction time is as long as 12 days. Therefore, the process is not economically reasonable.

In particular, when purifying the acetylated ester of formula I from the swine bile solid having 5-35 wt% of chenodeoxycholic acid content used in the present invention by using the above process, despite two times of recrystallization in ethanol solvent, the content of the final product is as low as 80%.

To overcome the above problems, the object of the present invention is to provide a new process for purifying the compound of formula I in high purity and yield, with with reducing the time required for the entire process.

EP 386 538 relates to a purification process of ursodeoxycholic acid (3-α-7-β-dihydroxycholic acid), and the deprotection step is carried out with 3-α-7-β-dihydrocholanic methyl ester.

US 3 919 266 discloses the deprotection of 3-α-7-α-dihydroxy-5-β-cholanic acid methyl ester by adding methanolic KOH, acidifying with aqueous HCl, and then extracting 3-α-7-α-dihydroxy-5-β-cholanic acid with ethyl ether and crystallizing the product from hot ethyl acetate.

### DISCLOSURE OF THE INVENTION

The present invention provides a process for purifying the compound of formula I, comprising the steps of: 1) pre-treatment of swine bile by dissolving swine bile solid derived from swine bile, containing the mixture of chenodeoxycholic acids of Formulae I-IV, 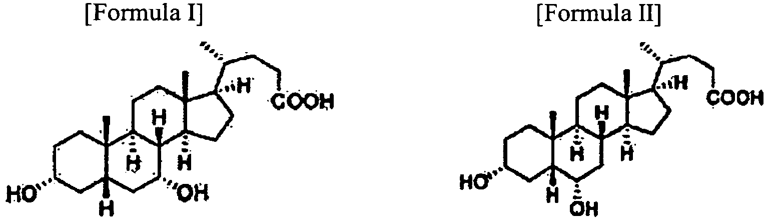 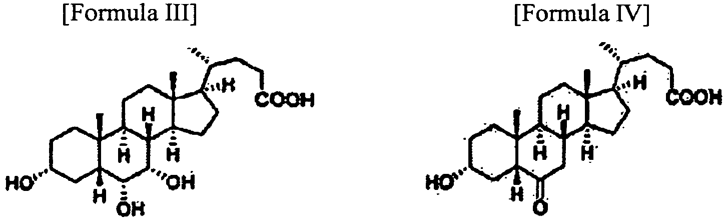 and having 5-35 wt% of chenodeoxycholic acid content, in organic solvent containing salt, wherein the organic solvent is ethyl acetate or acetone, and the salt is at least one selected from the group consisting of sodium chloride, anhydrous magnesium sulfate and anhydrous sodium sulfate; 2) esterification of a mixture of chenodeoxycholic acids of formulae I to IV by adding C₁₋₄ alcohol to the residue obtained from step (1); 3) acetylation of the mixture of esters of chenodeoxycholic acids of formulae I to IV by adding anhydrous acetic acid and a weak base selected from anhydrous sodium acetate or pyridine to the residue obtained from step (2); 4) adding non-polar organic solvent selected from hexane, heptane, octane or isooctane to the residue obtained from step (3) and stirring under reflux until all the residue is dissolved, and subsequently crystallizing and removing the acetylated esters of chenodeoxycholic acids of formulae III to IV, and a part of the acetylated ester of chenodeoxycholic acid of formula II; 5) adding methanol to the residue obtained from step (4) and crystallizing the compound of formula V 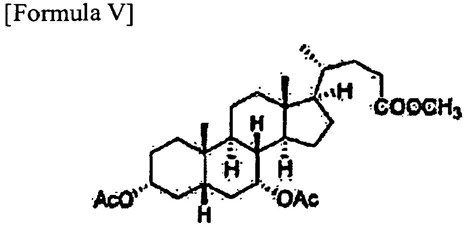 for 2~3 hours at the temperature range of 0 ~ 15°C; and 6) deprotection of the compound of formula V obtained from step (5) by adding water and base selected from sodium hydroxide or potassium hydroxide to the compound of formula V, and crystallization of the compound of formula I in the presence of water by adding acid selected from hydrochloric acid or sulfuric acid.

### DETAILED DESCRIPTION OF THE INVENTION

In the present specification, the phrase "swine bile solid" represents solid derived from swine bile, and contains the mixture of chenodeoxycholic acids of Formulae I~IV. 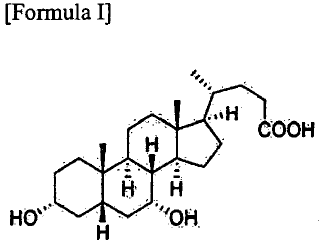 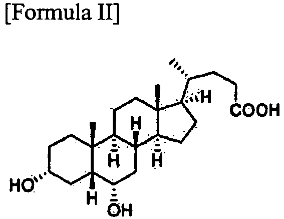 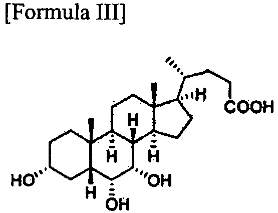 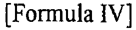 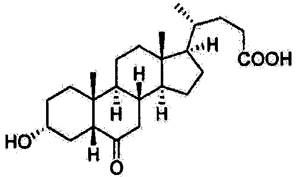 wherein, the compound of formula I represents chenodeoxycholic acid (3α,7α-dihydroxy-5β-cholic acid, CDCA); the compound of formula II represents hyodeoxycholic acid (3α,6α-dihydroxy-5β-cholic acid, HDCA); the compound of formula I represents hyocholic acid (3α,6α,7α-trihydroxy-5β-cholic acid, HCA); and the compound of formula IV represents 3α-hydroxy-6-oxo-5β-cholic acid(keto).

Hereinafter, each step for purifying chenodeoxycholic acid according to the present invention will be exemplified in detail.

### Step 1: Pre-treatment of swine bile solid

To use swine bile solid having 5~35 wt% of chenodeoxycholic acid content in the purification step, all of the swine bile solid is stirred and dissolved in organic solvent with reflux. Then, the mixture is cooled to room temperature, and more stirred for 1-2 hours. Then, insoluble materials are removed from the mixture with using filter paper, preferably filter paper and diatomaceous earth. The organic solvent is removed under reduced pressure to obtain residues (CDCA, HDCA, HCA and keto), which are used in the next step. Salt used in the present step can be optionally selected as long as it does not affect the compounds in the reactant. Preferably, the salt is at least one selected from the group consisting of sodium chloride, anhydrous magnesium sulfate (MgSO₄), and anhydrous sodium sulfate, more preferably sodium chloride. The amount of salt used in the present step is preferably 5~10 wt%, based on the amount of organic solvent. If the amount of salt is less than 5 wt%, water and insoluble materials (such as fatty acids, etc.) in the swine bile solid are not sufficiently removed, which makes the filtration difficult and reduces the yield and velocity of esterification reaction. If the amount of salt is more than 10 wt%, superfluous salt remains as impurity, which makes the purification difficult. Preferably, the organic solvent can be optionally selected from ones which can dissolve chenodeoxycholic acid of the swine bile solid and have no adverse effects thereto. More preferably, the solvent is ethyl acetate or acetone.

### Step 2: Esterification of chenodeoxycholic acid

Alcohol is added to the chenodeoxycholic acid mixture residue obtained from the prior step, and then the solution is stirred with reflux before the residue is completely dissolved. Then, the solution is cooled to 0~5°C. Acid catalyst is added to the solution, which is stirred with reflux at room temperature until the esterificaton reaction of chenodeoxycholic acid mixture is completed. When the reaction is completed, the solution is neutralized by adding base, and then filtered. The filtered material is washed with alcohol and concentrated under reduced pressure to obtain chenodeoxycholic acid ester mixture (CDCA-Me, HDCA-Me, HCA-Me and keto-Me) as residue. Alcohol used in the present step is not specifically limited, but preferably lower alcohol having 1~4 of carbon atoms, more preferably methanol, for easy esterification reaction. The acid catalyst used in the present step is preferably sulfuric acid or para-toluenesulfonic acid (PTSA), and the base is sodium bicarbonate, sodium carbonate or potassium carbonate.

### Step 3: Acetylation of chenodeoxycholic acid ester

All the hydroxy groups in chenodeoxycholic acid ester mixture are acetylated by adding anhydrous acetic and weak base to the residue obtained from the prior step with reflux. When the reaction is completed, toluene is added to the reaction solution with stirring at reflux. Then, the anhydrous acetic acid, acetic acid, and base remaining after the reaction are removed by concentrating the reaction solution under reduced pressure, to obtain a mixture of acetylated chenodeoxycholic acid ester (CDCA-diAc-Me, HDCA-diAc-Me, HCA-triAc-Me and keto-Ac-Me) as residue. The weak base used in the present step is preferably anhydrous sodium acetate or pyridine, more preferably anhydrous sodium acetate.

### Step 4: Removal of intermediate products of formulae III and IV

Non-polar solvent is added to the residue. The mixture is stirred with reflux until all the residue is dissolved and cooled to room temperature. With maintaining the temperature of solvent within 20~25°C, intermediate products of formulae III and IV; and a part of intermediate product of formula II (HCA-triAc-Me, keto-Ac-Me and part of HDCA-diAc-Me) are crystallized and removed by filtration. Thus filtered material is additionally washed with non-polar solvent, and then the filtered and washed solution is concentrated under reduced pressure, and dried in vacuum. The non-polar solvent used in the present step is preferably hexane, heptane, octane, isooctane and the like, more preferably hexane or heptane.

### Step 5: Production of chenodeoxycholic acid-diacetate-methyl-ester

To produce chenodeoxycholic acid-diacetate-methyl-ester(CDCA-diAc-Me) of formula V which is an intermediate product for preparing the compound of formula I, alcohol solvent is added to the product obtained from the prior step, and then the compound of formula V is crystallized by standing the mixture for 2-3 hours at 0°C ~15°C, preferably 0°C~5°C.

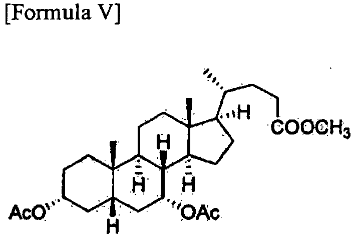

If the temperature is less than 0°C, the content for the compound of formula V decreases, and if the temperature is more than 15°C, the crystallization is not done sufficiently. When the compound of formula V is crystallized, the intermediate product of formula II is removed from the solvent by using filtration. Thus filtered material is washed with alcohol solvent, and dried in vacuum to obtain chenodeoxycholic acid-diacetate-methyl-ester of formula V as crude product. To purify the compound of formula V in high purity, recrystallization is performed until the content for the compound of formula V becomes 98.5% or more, preferably 99% or more, under the same conditions. To obtain the content of 99% or more, it is preferable to perform the recrystallizaton three (3) times or more. The alcohol used in the crystallization is methanol, considering the content for the compound of formula V. The amount of alcohol used in the crystallization is 0.5-3 times, preferably 1.5-3 times, to the amount of residue. If the amount is less than 0.5 times, the filtration is difficult since crystals coagulate each other. If the amount becomes more than 3 times, the content for the compound of formula V is not affected by the amount.

### Step 6: Deprotection and crystallization of chenodeoxycholic acid

The compound of formula V obtained from the prior step is deprotected in the presence of base, and the pH of the reaction solution is adjusted to 4 or lower, preferably 2~3 in acid condition, to form chenodeoxycholic acid of formula I. Simultaneously, the reaction solution stands at 35~45°C, preferably 35~40°C, to crystallize the compound of formula I in the presence of water. The reaction solution is filtered, washed with water, and dried in vacuum to refine chenodeoxycholic acid purely. The base used for the deprotection is not specifically limited, but sodium hydroxide or potassium hydroxide is preferred for the post-treatment step. If the pH is more than 4, crystals are not formed, and if the pH is less than 2, the purity of the final product is reduced due to superfluous acid. If the crystallization temperature in water is less than 35°C, the purity of the compound of formula I decreases, and if the temperature is more than 45°C, the filtration is difficult since crystals derived from the compound of formula I coagulate each other. The acid used in neutralizing the reaction solution is also not specifically limited, but hydrochloric acid or sulfuric acid is preferred for the post-treatment step. Since the residue obtained from the prior step contains 98.5% or more of the compound of formula V, preferably 99% or more, the compound of formula I can be purely refined without additional crystallization using organic solvent since the content of impurities is low. The product containing the compound of formula I crystallized in water according to the present invention is suitable for industrial manufacturing process since its melting point is about 20°C higher, and has lower volume, than crystallized compound of formula I in organic solvent.

The present invention will be more specifically explained in the following examples. However, it should be understood that the following examples are intended to illustrate the present invention, and cannot limit the scope of the present invention in any manner.

### Analytical method

HPLC was used to confirm the intermediate products separated form each step, and the test conditions are as follows:
Column: Capcell pak UG120 C18 (4.6 X 250mm, Shiseido)
Mobile phase: acetonitrile/water (85:15)
Detector: ultraviolet spectrometer (210nm)
Flow rate: 1.0ml/min
Insertion: 20µℓ

### Example

### Step 1: Pre-treatment of swine bile solid

150g of swine bile solid having 30~35 wt% of chenodeoxycholic acid content, and 60g of sodium chloride in 600 ml of ethyl acetate were stirred with reflux for 1 hour, to dissolve all the swine bile solid. Then, the mixture was cooled to 20~25°C, stirred for 1 hour, and filtered through diatomaceous earth, and thus filtered material was washed with 60 ml of ethyl acetate. Organic solvent was removed by concentrating the filtered material under reduced pressure to obtain chenodeoxycholic acid mixture (CDCA, HDCA, HCA and keto) as residue.

### Step 2: Esterification of chenodeoxycholic acid

To the residue obtained from the prior step was added 375ml of methanol, and the mixed solution was stirred with reflux for 30 minutes until the residue was completely dissolved. This solution was cooled to 0~10°C, 4.88ml of sulfuric acid was added to the solution with stirring, and then the esterification reaction of chenodeoxycholic acid mixture was completed by stirring at 20~22°C for 2 hours. When the esterification reaction is completed, the solution was neutralized with 53.9g of sodium bicarbonate, and then filtered. Thus filtered material was washed with 150ml of methanol, and concentrated under reduced pressure to obtain 134g of chenodeoxycholic acid ester mixture (CDCA-Me, HDCA-Me, HCA-Me and keto-Me) as residue.

### Step 3: Acetylation of chenodeoxycholic acid ester

To 134g of chenodeoxycholic acid ester mixture obtained from the prior step were added 20g of anhydrous sodium acetate and 200ml of anhydrous acetic acid. The mixed solution was refluxed at 120~140°C for 5 hours, and then immediately concentrated under reduced pressure. Anhydrous acetic acid and acetic acid were completely removed by adding 25ml of toluene to the reaction solution, stirring with reflux for 15 minutes, and concentrating under reduced pressure, to obtain acetylated chenodeoxycholic acid ester mixture (CDCA-diAc-Me, HDCA-diAc-Me, HCA-triAc-Me and keto-Ac-Me) as residue. HPLC result for the residue (RT): HCA-triAc-Me (8.76min), keto-Ac-Me (9.05min), CDCA-diAc-Me (12.21min), and HDCA-diAc-Me (12.81min)

### Step 4: Removal of intermediate products of formulae III and IV

To the residue obtained from the prior step was added non-polar solvent (400ml of hexane), and then the mixed solution was stirred with reflux for 30 minutes. Then, hexane solvent was cooled to 25~35°C, stirred for 3 hours, and then filtered. Thus filtered material (HCA-triAc-Me, keto-Ac-Me and part of HDCA-diAc-Me) was additionally washed with 65ml of hexane, and the filtered and washed solution was concentrated under reduced pressure to obtain the intermediate products of formulae I and II (CDCA-diAc-Me, HDCA-diAc-Me) as residue. HPLC result for the residue (RT): CDCA-diAc-Me (12.21min) and HDCA-diAc-Me (12.81min).

### Step 5: Production of chenodeoxycholic acid-diacetate-methyl-ester

To the residue obtained from the prior step was added 270ml of methanol, and the mixed solution is stirred with reflux for 30 minutes, cooled to 0~10°C, more stirred for 2 hours, and then filtered. The filtered material (CDCA-diAc-Me) was washed with methanol 70ml, and dried in vacuum at 60°C to obtain 85% content of crude product. Then, to the crude product was added 72ml of methanol, and recrystallization was performed to the mixture at 0~5°C for 2 hours. Recrystallization is additionally performed to the mixture one more time to obtain 99% content of chenodeoxycholic acid-diacetate-ester. The yield is 24.5g (19.5g+mother liquor 5g). m.p.: 128~129°C. HPLC result for the residue (RT): CDCA-diAc-Me (12.21 min).

### Step 6: Deprotection and crystallization of chenodeoxycholic acid

To 220ml of water were added 24.5g of chenodeoxycholic acid-diacetate-ester and 29.5g of sodium hydroxide, and then the solution was stirred with reflux for 4 hours. To the solution was added 370ml of water. The solution's pH is adjusted to 2.0-3.0 by using 59ml of hydrochloric acid. Then, the solution was stirred at 35~45°C for 1 hour, and then filtered. The filtered material was washed with 24.5ml of water and dried in vacuum at 70°C to obtain 19.5g of pure chenodeoxycholic acid. m.p.: 160~161°C, [α]_{D}²⁵ +13.0°(c=1, CHCl₃).

### Comparative Example

### Step 1: Pre-treatment of bile

150g of concentrated swine bile was dissolved in 1000ml of hot water. Then, 100g of sodium hydroxide was added, and the solution was stirred with reflux for 20 hours. This solution was cooled to 25°C. 1500ml of water was added to the solution, which was kept cool for one day. 10g of diatomaceous earth was added to the reaction solution, which was then stirred and filtered to remove precipitated sodium 3α-hydroxy-6-ketocholate of formula IV. The filtrate was adjusted to pH 8 by using conc. sulfuric acid, and then stirred for 15 minutes after adding 5g of sodium hydrosulfite. Then, 400ml of ethyl acetate was added to the solution, which was then adjusted to pH 5 by using diluted sulfuric acid. The solution was stirred for 30 minutes, and aqueous layer was removed therefrom by layer separation. To the organic layer were added 7g of diatomaceous earth and 7g of active carbon, which was then stirred for 30 minutes and filtered. Thus filtered material was washed with 50ml of ethyl acetate, and concentrated under reduced pressure.

### Step 2: Esterfication of bile acid

The residue obtained from the prior step was dissolved in 300ml of methanol. Then, the solution was neutralized with sodium bicarbonate (pH 7), filtered, and then concentrated under reduced pressure.

### Step 3: Removal of methyl ester of formula II

The residue obtained from the prior step was dissolved in 320ml of hot benzene, and the mixed solution was concentrated to 225ml, and kept cold for one day. Then, the solution was filtered; thus filtered material (methyl ester benzene adduct of formula II) was washed with benzene, and the benzene-filtered and washed solution was concentrated under reduced pressure.

### Step 4: Acetylation of bile acid ester

To the residue obtained from the prior step were added 75ml of anhydrous acetic acid and 7.5g of anhydrous sodium acetate, and then the mixed solution was stirred with reflux for 5 hours. The remaining anhydrous acetic acid was removed by distilling anhydrous acetic acid, stirring with reflux for 15 minutes after adding 35ml of methanol, and then distilling under reduced pressure.

### Step 5: Removal of acetylated ester of formula III

The residue obtained form the prior step was refluxed in 200ml of hexane solvent, and the solution was stored at 20°C for one day, and filtered. Thus filtered material (crude crystal of HDCA-triAc-Me) was washed with hexane, and the filtered and washed solution was distilled under reduced pressure.

### Step 6: Separation of the compound of formula V

The residue obtained form the prior step was dissolved in 46ml of hot ethanol, and then kept cool for one day. This solution was filtered, and thus filtered material was washed with 27ml of cold ethanol, and dried in vacuum at 60°C. 21.5g of the compound of formula V was recrystallized 3 times by using ethanol to obtain 18.5g of product. m.p. 119~121°C; [α]_{D}²⁵ +10.4°(c=1, Dioxane); [α]_{D}²⁵ +13.8°(c=1, CHCl₃).

### Step 7: Saponification and neutralization

To 185ml of water were added 18.5g of chenodeoxycholic acid-diacetate-methylester and 18.5g of sodium hydroxide, and then the mixed solution was stirred with reflux for 14 hours. Then, the solution's pH was adjusted to 4.5 by using conc. sulfuric acid.

### Step 8: Production of the compound of formula I

The reaction solution was extracted by using ethyl acetate, and aqueous layer was discarded therefrom. Ethyl acetate layer in the solution was washed with 6% saline, and the solution was distilled to about 90ml. This solution was cooled, kept cool for one day after adding 90ml of hexane, and filtered. Thus filtered material was washed with 20ml of hexane, and dried in vacuum at 60°C to produce 12.7g of chenodeoxycholic acid. m.p. 142~145°C; [α]_{D}²⁵ +13.0°(c=1, CHCl₃).

### INDUSTRIAL APPLICABILITY

The present invention can purify chenodeoxycholic acid of formula I from swine bile solid in high yield and purity. Also, the present invention is suitable for industrial purification by reducing the purification time.

## Claims

1. A process for purifying the compound of formula I, comprising the steps of:
1) pre-treatment of swine bile by dissolving swine bile solid derived from swine bile, containing the mixture of chenodeoxycholic acids of Formulae I-IV, 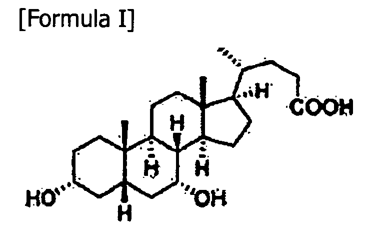 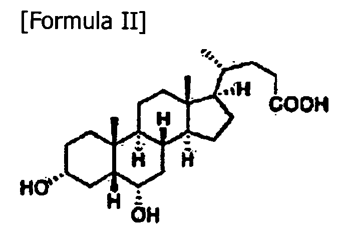 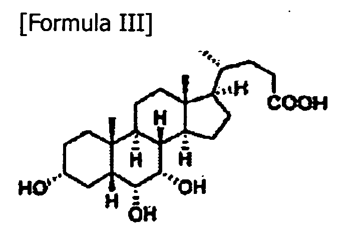 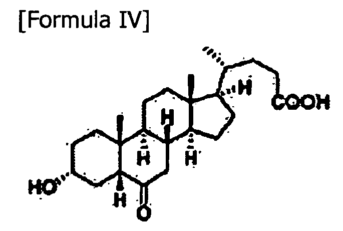 and having 5-35 wt% of chenodeoxycholic acid content, in organic solvent containing salt, wherein the organic solvent is ethyl acetate or acetone, and the salt is at least one selected from the group consisting of sodium chloride, anhydrous magnesium sulfate and anhydrous sodium sulfate;
2) esterification of a mixture of chenodeoxycholic acids of formulae I to IV by adding C₁₋₄ alcohol to the residue obtained from step (1);
3) acetylation of the mixture of esters of chenodeoxycholic acids of formulae I to IV by adding anhydrous acetic acid and a weak base selected from anhydrous sodium acetate or pyridine to the residue obtained from step (2);
4) adding non-polar organic solvent selected from hexane, heptane, octane or isooctane to the residue obtained from step (3) and stirring under reflux until all the residue is dissolved, and subsequently crystallizing and removing the acetylated esters of chenodeoxycholic acids of formulae III to IV, and a part of the acetylated ester of chenodeoxycholic acid of formula II;
5) adding methanol to the residue obtained from step (4) and crystallizing the compound of formula V 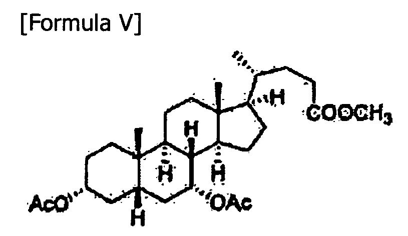 for 2~3 hours at the temperature range of 0 ~ 15°C; and
6) deprotection of the compound of formula V obtained from step (5) by adding water and base selected from sodium hydroxide or potassium hydroxide to the compound of formula V, and crystallization of the compound of formula I in the presence of water by adding acid selected from hydrochloric acid or sulfuric acid.

2. The process according to claim 1, wherein the amount of salt is 5 - 10 wt%, based on the total weight of organic solvent.

3. The process according to claim 1, wherein the purity of the compound of formula V obtained from step (5) is 98.5% or more.

4. The process according to claim 1, wherein step (5) is carried out in the temperature range of 0 ~ 5°C.

5. The process according to claim 1, wherein the amount of methanol used in step (5) is 0,5 ~ 3 times the amount of residue obtained from step (4).

6. The process according to claim 1, wherein the pH of the deprotection in step (6) is 4 or less.

7. The process according to claim 1, wherein the crystallization in the presence of water in step (6) is carried out in the temperature range of 35 ~ 45°C.

## Patentansprüche

1. Verfahren zum Reinigen der Verbindung der Formel I, umfassend die Schritte:
1) Vorbehandlung von Schweinegallenflüssigkeit durch Lösen von aus Schweinegallenflüssigkeit abgeleiteten Schweinegallenfeststoff, enthaltend das Gemisch von Chenodesoxycholsäuren der Formeln I-IV, 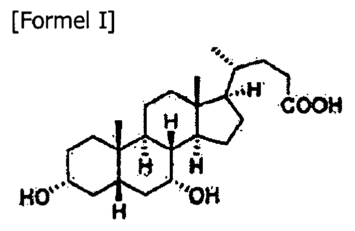 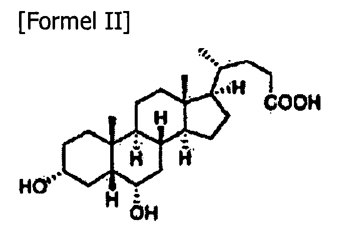 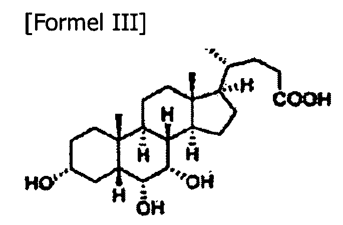 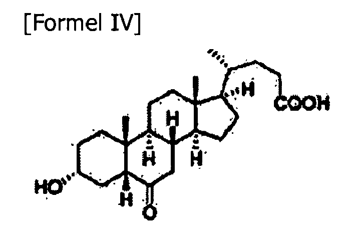 und mit einem 5-35 Gew.-% Chenodesoxycholsäuregehalt, in Salz enthaltendem organischen Lösungsmittel, wobei das organische Lösungsmittel Ethylacetat oder Aceton ist, und das Salz mindestens eines ist, ausgewählt aus der Gruppe, bestehend aus Natriumchlorid, wasserfreiem Magnesiumsulfat und wasserfreiem Natriumsulfat;
2) Veresterung eines Gemischs von Chenodesoxycholsäuren der Formeln I bis IV durch Hinzufügen von C₁₋₄-Alkohol zu dem aus Schritt (1) erhaltenen Rest;
3) Acetylierung des Gemischs von Estern von Chenodesoxycholsäuren der Formeln I bis IV durch Hinzufügen von wasserfreier Essigsäure und einer schwachen Base, ausgewählt aus wasserfreiem Natriumacetat oder Pyridin zu dem aus Schritt (2) erhaltenen Rest;
4) Hinzufügen von nicht-polarem organischen Lösungsmittel, ausgewählt aus Hexan, Heptan, Oktan, oder Isooktan zu dem aus Schritt (3) erhaltenen Rest und Rühren unter Rückfluss bis der Rest gelöst ist, und anschließend Kristallisieren und Entfernen der acetylierten Ester von Chenodesoxycholsäuren der Formeln III bis IV und eines Teils des acetylierten Esters von Chenodesoxycholsäure der Formel II;
5) Hinzufügen von Methanol zu dem aus Schritt (4) erhaltenen Rest und Kristallisieren der Verbindung der Formel V 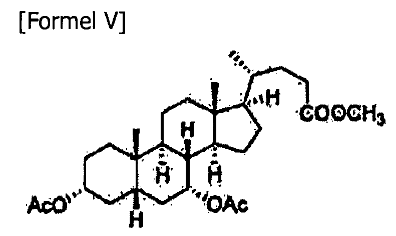 für 2~3 Stunden bei dem Temperaturbereich von 0 ~ 15°C; und
6) Entschützen der aus Schritt (5) erhaltenen Verbindung der Formel V durch Hinzufügen von Wasser und Base, ausgewählt aus Natriumhydroxid und Kaliumhydroxid zu der Verbindung der Formel V, und Kristallisierung der Verbindung der Formel I in der Gegenwart von Wasser durch Hinzufügen von Säure, ausgewählt aus Salzsäure oder Schwefelsäure.

2. Verfahren nach Anspruch 1, wobei die Menge an Salz 5-10 Gew.-% beträgt, basierend auf dem Gesamtgewicht an organischem Lösungsmittel.

3. Verfahren nach Anspruch 1, wobei die Reinheit der aus Schritt (5) erhaltenen Verbindung der Formel V 98,5 % oder mehr beträgt.

4. Verfahren nach Anspruch 1, wobei Schritt (5) in dem Temperaturbereich von 0 ~ 5°C durchgeführt wird.

5. Verfahren nach Anspruch 1, wobei die Menge an in Schritt (5) verwendetem Methanol 0,5 ~ 3 mal die Menge an aus Schritt (4) erhaltenem Rest ist.

6. Verfahren nach Anspruch 1, wobei der pH der Entschützung in Schritt (6) 4 oder weniger beträgt.

7. Verfahren nach Anspruch 1, wobei die Kristallisierung in der Gegenwart von Wasser in Schritt (6) in dem Temperaturbereich von 35 ~ 45°C durchgeführt wird.

## Revendications

1. Procédé de purification du composé de formule I, comprenant les étapes de :
1) pré-traitement de bile porcine par dissolution de bile porcine solide dérivée de bile porcine, contenant le mélange d'acides chénodésoxycholiques de formules I à IV, 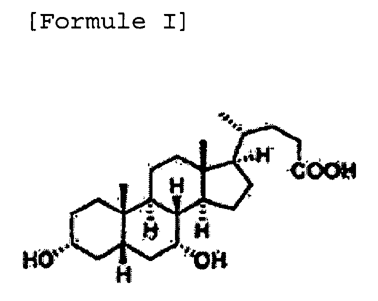 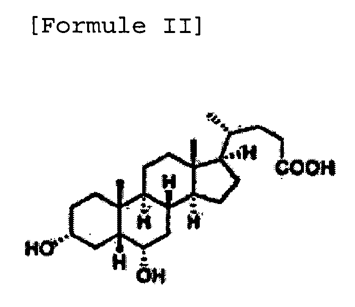 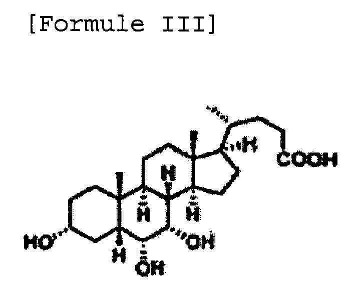 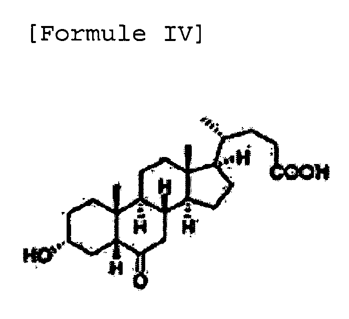 et ayant une teneur en acide chénodésoxycholique de 5 à 35 % en poids, dans un solvant organique contenant un sel, dans lequel le solvant organique est l'acétate d'éthyle ou l'acétone, et le sel est au moins un sel choisi dans le groupe constitué par le chlorure de sodium, le sulfate de magnésium anhydre et le sulfate de sodium anhydre ;
2) estérification d'un mélange d'acides chénodésoxycholiques de formules I à IV par l'ajout d'un alcool en C₁₋₄ au résidu obtenu à partir de l'étape (1) ;
3) acétylation du mélange d'esters d'acides chénodésoxycholiques de formules I à IV par l'ajout d'acide acétique anhydre et d'une base faible choisie parmi l'acétate de sodium anhydre ou la pyridine au résidu obtenu à partir de l'étape (2) ;
4) ajout d'un solvant organique non polaire choisi parmi l'hexane, l'heptane, l'octane ou l'isooctane au résidu obtenu à partir de l'étape (3) et agitation sous reflux jusqu'à ce que le résidu soit dissout, et par la suite cristallisation et élimination des esters acétylés d'acides chénodésoxycholiques de formules III à IV, et d'une partie de l'ester acétylé de l'acide chénodésoxycholique de formule II ;
5) ajout de méthanol au résidu obtenu à partir de l'étape (4) et cristallisation du composé de formule V 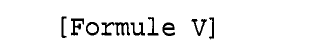 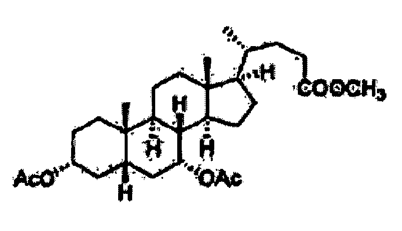 pendant 2 à 3 heures à la plage de températures de 0 à 15°C ; et
6) déprotection du composé de formule V obtenu à partir de l'étape (5) par ajout d'eau et d'une base choisie parmi l'hydroxyde de sodium ou l'hydroxyde de potassium au composé de formule V, et cristallisation du composé de formule I en présence d'eau par ajout d'un acide choisi parmi l'acide chlorhydrique ou l'acide sulfurique.

2. Procédé selon la revendication 1, dans lequel la quantité de sel est de 5 à 10 % en poids, par rapport au poids total de solvant organique.

3. Procédé selon la revendication 1, dans lequel la pureté du composé de formule V obtenu à partir de l'étape (5) est de 98,5 % ou plus.

4. Procédé selon la revendication 1, dans lequel l'étape (5) est réalisée dans la plage de température de 0 à 5°C.

5. Procédé selon la revendication 1, dans lequel la quantité de méthanol utilisé à l'étape (5) est de 0,5 à 3 fois la quantité de résidu obtenu à l'étape (4).

6. Procédé selon revendication 1, dans lequel le pH de la déprotection à l'étape (6) est de 4 ou moins.

7. Procédé selon la revendication 1, dans lequel la cristallisation en présence d'eau à l'étape (6) est réalisée dans la plage de température de 35 à 45°C.
